Europäisches Patentamt

European Patent Office

Office européen des brevets

(19)

(11) Publication number: **0 134 290**
A1

(12)

# EUROPEAN PATENT APPLICATION

(21) Application number: **83108714.3**

(22) Date of filing: **05.09.83**

(51) Int. Cl.⁴: **A 61 K 9/22**
**A 61 K 31/52**

(43) Date of publication of application:
20.03.85 Bulletin 85/12

(84) Designated Contracting States:
AT BE CH DE FR GB IT LI LU NL SE

(71) Applicant: VEREX LABORATORIES, INC.
5241 South Quebec
Englewood Colorado 80111(US)

(72) Inventor: Dunn, James Michael
3236 Hinsdale Place
Littleton Colorado 80122(US)

(72) Inventor: Haas, Ronald Thomas
8413 S. Painted Sky Street
Highland Park Colorado 80126(US)

(74) Representative: Modiano, Guido et al,
MODIANO & ASSOCIATI S.A.S. Via Meravigli, 16
I-20123 Milan(IT)

(54) Constant release rate solid dosage theophyllineformulation.

(57) A constant release rate theophylline formulation in tablet unit dosage form, said tablet comprising an intimate admixture of from 100 to 350 mg of theophylline, from about 4.5 to 12.7 weight percent of a slow-dissolving water-insoluble cellulose derivative, and from about 35 to 75 weight percent of a pharmaceutically acceptable bulking agent or diluent, and a lubricant.

CONSTANT RELEASE RATE SOLID DOSAGE
THEOPHYLLINE FORMULATION


The present invention relates to an improved pharmaceutical formulation for theophylline and more particulary to a solid dosage, constant release rate theophylline formulation.

Theophylline and its salts and derivatives are sold in varying dosage forms for symptomatic treatment of bronchial asthma and reversible bronchospasm which may occur in association with chronic bronchitis, emphysema, and other obstructive bronchial diseases. The drug relieves the primary symptoms of asthma, including the shortness of breath, wheezing and dyspnea improves pulmonary function as measured by increased flow rates and vital capacity.

There are a number of commercially available forms of the widely used drug, i.e. Elixophyllin® (Cooper), Somophyllin® (Fisons), Theodur® (Key), Theolaire® (Riker) and Theophyl® (Knoll), to name a few. In addition, there are numerous combination products containing, for example, theophylline, ephedrine hydrochloride and phenobarbital (Tedral® sold by Mead Johnson), theophylline, ephedrine sulfate, pheonbarbital and guaifenesin (Bronkotabs®, Bronkolixir® (Breon), theophylline, calcium salicilate and potassium iodide (Theokin® sold by Knoll), etc.

Conventional theophylline preparations generally have to be taken every four to six hours. Because of the problems encountered with patient compliance when medicine administered on a chronic basis has to be taken frequently, attention has been directed to the formulation of longer-acting theophylline preparations. One of the most successful sustained release theophylline preparations has been the preparation sold by Key Pharmaceuticals, Inc. under the trademark Theodur.

The present invention provides a constant release rate theophyllilne formulation which is administered once or twice a day to provide prophylactic treatment of bronchial asthma.

The present invention provides a constant release rate theophylline tablet which may be administered once or twice daily to control the symptoms of bronchial asthma and other chronic respiratory disease. The constant release rate tablet contains a therapeutically effective amount of theophylline admixed with a slow-dissolving, water insoluble cellulose derivative and a tablet excipient. Coloring agents or dyes as well as other bulking agents and a tablet lubricant can also be included.

The constant release rate theophylline tablet of the present invention comprises an intimate admixture of a therapeutically effective amount of theophylline, from about 4.5 to 12.7, and preferably 5.1 to 10.3 weight percent of a slow-dissolving, water insoluble cellulose derivative, such as cellulose acetate phthalate, ethyl cellulose and the like, and from 35 to 75 weight percent of a bulking excipient. In addition, the tablet may include a coloring agent or dye, lubricants and the like.

Preferably, each tablet contains from 100-350 mg of theophylline.

The preferred slow-dissolving water insoluble cellulose derivates are cellulose acetate phthalate and ethyl cellulose alone, or in combination with each other. Other such derivatives are well known in the art and include methyl cellulose, powdered cellulose, hydroxyethyl cellulose, hydroxypropyl cellulose, hydroxypropylmethyl cellulose, microcrystallline cellulose and the like.

Dibasic calcium phosphate is the preferred bulking excipient.

The term "constant release rate," as used herein, refers to a formulation wherein the *in vitro* release of theophylline from the tablet, using a U.S.P. dissolution apparatus, pH 7.5, is constant and linear against time until all of the drug is released when plotted on an x,y graplh using the formula

$$K = \frac{dc}{dt}$$

wherein K = constant, dc = decreasing concentration and dt = decreasing time, a straight line is formed. Calculation of the data points by linear regression analysis should give a value of 0.85 to 1.0. A value of 1.0 is a perfect straight line.

The preparation of the theophylline tablets of the present invention is simple, straight-forward and results in a significant savings in production costs, and hence costs to the patient, over presently available capsule formulations.

Generally speaking, constant release rate theophylline tablets are advantageously prepared by dissolving the slow-dissolving, water insoluble cellulose derivative in a suitable solvent and carefully admixing the solution with a dry blended powder of theophylline, and excipient to form a wet granulation. The granulation is dried, screened through a 14-20 mesh screen, and the sized granules are then compressed in a rotary or single station punch press. For ease of tableting, it is preferred to add a lubricant such as magnesium stearate to the dried granules prior to tabletting.

The preferred solvent is a 1:1[v/v] mixture of isopropyl alcohol and methylene chloride. Other suitable solvents include, but are not limited to, lower aliphatic alcohols such as methanol, n-propanol, ethanol, denatured alcohol, acetone, methyl ethyl acetate and non-chlorinated hydrocarbons.

Turning to the preferred process of the present invention, the cellulose derivative, preferably cellulose acetate phthalate, is dissolved and dispersed in a solvent, preferably isopropyl alcohol-methylene chloride [1:1 (v/v)], which is used to make a wet granulation. The amount of cellulose derivative may range from 4.5-12.7 weight percent of the final tablet weight, and preferably from 5-10.3 weight percent. Generally from about 60 to 20 weight percent of solvent, based on the total weight of the formulation, and preferably from about 50 to 35 weight percent is employed.

The cellulose derivative and solvent are blended until the cellulose derivative is completely dispersed and clarity of solution has been obtained. Generally, it takes between 35-40 minutes per 125 liters of fluid, using a lightening blender, to effects solution.

The granulating fluid is then added to dry blended U.S.P. anhydrous theophyllilne, 16-200 mesh and preferably 40-80 mesh, and a bulking agent, in a slow, steady stream, preferably at a rate no faster than 3 minutes per liter of solution. If the fluid is added too rapidly, it is difficult to obtain an even granulation.

Following the addition of the cellulose solution, blending is continued for from about 3 to 12 minutes, preferably 3.5 to 6 minutes per kilogram of material.

It is critical that the wet granulation be completely dried before screening. Failure to observe this technique may result in rupture of the granules and a loss of the constant release rate profile. In conventional prior art tableting procedures, the wet granulations are generally screened immediately after formation and then dried. However, if the prior art processes are employed, the constant release rate profile of the theophylline tablets of the present invention may be destroyed.

It is preferable to maintain the humidity below 50% both during the granulating and tableting processes.

Because of the unique and superior constant release rate properties, theophylline tablets made by the described process and ingredients can be administered once or possibly twice a day to provide 24 hour suppression of wheezing, bronchospasm and dyspnea.

### Example 1

Theophylline tablets weighing 665 mg and containing 300 mg of theophylline were prepared from the following formulation and by the following process.

| Ingredient | Amount |
|---|---|
| Theophyllilne, anhydrous | 300 gm |
| Dibasic Calcium Phosphate | 300 gm |
| Cellulose Acetate Phthalate | 30 gm |
| Ethyl Cellulose | 30 gm |
| Isopropyl Alcohol | 150 ml |
| Methylene Chloride | 150 ml |
| Magnesium Stearate | 4.6 gm |

The theophyllilne and dibasic calcium phosphate were dry blended in a Hobart mixer. The dissolved cellulose acetate phthalate was slowly added until a wet granulation was formed. The material was discharged onto paper-lined trays and dried overnight. The granules were then screened through a number 16 screen and blended with magnesium stearate. The granulate was then compressed into tablets using a rotary press.  Each tablet weighed 665 mg and contained 300 mg  of theophylline with a tablet hardness of 10 kp.

## Example 2

To demonstrate that the product does not have a tendency to dissolve in the stomach the following disintegration test was done using tablets made from Example 1.

### Table 1

% Tablets Residue After 6 Hours

Disintegration, U.S.P. Basket Apparatus, No 2

Disks

#### Gastric Acid pH 1.2

| | Start Weight mg | 6 hours weight mg | % Residue Remaining 6 hours |
|---|---|---|---|
| 1. | 667 | 633 | 94.9% |
| 2. | 664 | 630 | 94.9% |
| 3. | 660 | 629 | 95.3% |
| 4. | 665 | 652 | 98.1% |
| 5. | 668 | 638 | 99.6% |
| 6. | 661 | 640 | 96.8% |
| | ---- | --- | ----- |
| | 664 | 637 | 96.8% |

### Table 2

#### Intestinal Fluid pH 7.5

| | Start Weight mg | 6 hours weight mg | % Residue Remaining 6 hours |
|---|---|---|---|
| 1. | 665 | 199.5 | 30.8% |
| 2. | 668 | 201.3 | 30.1% |
| 3. | 664 | 182.4 | 27.5% |
| 4. | 661 | 197.5 | 29.9% |
| 5. | 665 | 202.7 | 30.5% |
| 6. | 667 | 204.6 | 30.7% |
| | ---- | ----- | ----- |
| mean | 665 | 198 | 29.5% |

These studies suggest that the theophylline tablets made by the present invention are relatively resistant to disintegration in an acid media. In a neutral pH which is found beyond the stomach in the small intestine a slow, steady erosion of the tablet system is noted.

## Example 3

While the tablets showed a steady constant erosion, to be considered a true constant release rate formulation requires testing by dissolution. Tablets made by Example 1 were placed in an approved U.S.P. dissolution apparatus and assayed for theophylline content. Twenty tablets were randomly weighed and the average tablet weight was 663 mg. The amount of theophylline per tablet was reported as 290.4 mg. Dissolution was carried out using U.S.P. apparatus II, pH 7.5 150 RPM at 37 C.

### % Drug Released

| Tablet No. | 1 hour | 2 hour | 3 hour | 4 hour |
|---|---|---|---|---|
| 1 | 25.99 | 45.41 | 59.51 | 70.06 |
| 2 | 22.17 | 33.16 | 41.99 | 54.21 |
| 3 | 23.75 | 35.85 | 43.36 | 52.08 |
| 4 | 26.28 | 45.99 | 64.57 | 74.74 |
| 5 | 27.40 | 44.55 | 58.97 | 68.74 |
| 6 | 36.75 | 48.14 | 57.03 | 66.56 |
| mean | 27.06 | 42.18 | 54.24 | 64.40 |

Linear regression analysis of mean % drug released shows an r value of 0.996 which is graphically demonstrated in Figure 1.

This data demonstrates that tablets made from the present formulation have a constant release rate profile and have little disintegration or tablet disruption in gastric acid.

## Example 4

A single dose bioavailability study was performed in two healthy male volunteers weighing 80 and 72.2 kg, respectively. After a complete medical evaluation, the two subjects were given a single oral dose (300 mg, tablet of example 1) of theophylline formulated as described in Example 1. The subjects ingested the theophylline tablets with 250 ml of water and blood samples were drawn at the time indicated. Plasma theophylline was measured by high pressure liquid chromatography. The results of this analysis at the specified time intervals are shown below in Table 3.

### Table 3
#### Time After Dose
Plasma Theophylline in mcg/ml

| | 0.5 | 1hr | 2hr | 4hr | 6hr | 8hr | 10hr | 12hr | 16hr | 24hr |
|---|---|---|---|---|---|---|---|---|---|---|
| #1 | .69 | 1.23 | 2.48 | 3.57 | 3.24 | 2.94 | 2.47 | 2.14 | 1.79 | 1.23 |
| #2 | .49 | 1.18 | 2.02 | 3.15 | 3.42 | 3.32 | 2.91 | 2.50 | 2.10 | 1.15 |
| Mean | .59 | 1.21 | 2.25 | 3.36 | 3.33 | 3.13 | 2.69 | 2.32 | 1.95 | 1.19 |

The alpha slope is that time of absorption and distribution in a biphasic curve.

The beta or excretion phase is the elimination or plasma dwell time. Calculation for the beta slope is as follows:

| Subject #1 | Subject #2 |
|---|---|
| correlation=0.970 | correlation=0.993 |
| slope        =1.173 | slope        =1.733 |
| intercept  =0.463 | intercept  =0.857 |

The alpha slope is that time of absorption and distribution in a biphasic curve.

The beta or excretion phase is the elimination or plasma dwell time. Calculation for the beta slope is as follows:

| Subject #1 | Subject #2 |
|---|---|
| correlation=0.970 | correlation=0.933 |
| slope        =7.951 | slope        =7.601 |
| intercept =31.169 | intercept =32.175 |
| t 1/2 = 16.94 hrs | t 1/2 = 19.178 |

Calculation of these same parameters of the mean plasma theophylline levels gives the following:

| Alpha Slope | Beta Slope |
|---|---|
| correlation = 0.983 | correlation = 0.987 |
| slope = 1.250 | slope = 8.351 |
| intercept = 0.443 | intercept = 32.868 |
| | t 1/2 = 18.837 hrs. |

These figures can be compared to a similar dose of regular theophylline which has a published excretion half life of 9.0 [+- 2.1 hours].

It would appear that theophylline prepared by the presentinvention has a plasma dwell time which is approximately two times greater than theophylline prepared in the usual manner.

## Example 4

Theophylline tablets weighing 654 mg and containing 300 mg of theophylline were prepared from the following formulation and by the following process.

| Ingredient | Amount |
|---|---|
| Theophylline, anhydrous | 300 gm |
| Dibasic Calcium Phosphate | 300 gm |
| Cellulose Acetate Phthalate | 45 gm |
| Ethyl Cellulose | 5 gm |
| Isopropyl Alcohol | 125 ml |
| Methylene Chloride | 125 ml |
| Magnesium Stearate | 4.0gm |

The theophylline and dibasic calcium phosphate were dry blended in a Hobart mixer. The dissolved cellulose acetate phthalate was slowly added until a wet granulation was formed. The material was discharged onto paper-lined trays and dried overnight. The granules were then screened through a number 16 screen and blended with magnesium stearate. The granulate was then compressed into tablets using a rotary press. Each tablet weighed 654 mg and contained 300 mg of theophylline with a tablet hardness of 10 kp.

0134290

CLAIMS:

1. A constant release rate theophylline formulation in tablet unit dosage form, said tablet comprising an intimate admixture of from 100 to 350 mg of theophylline, from about 4.5 to 12.7 weight percent of a slow-dissolving, water-soluble cellulose derivative, from about 35 to 75 weight percent of a pharmaceutically acceptable bulking agent or a diluent and a lubricant.

2. The theophylline tablet of Claim 1 wherein said water-insoluble cellulose derivative is cellulose acetate phthalate.

3. The theophyline tablet of Claim 1 wherein said water-insoluble cellulose derivative is a mixture of cellulose acetate phthalate and ethyl cellulose.

4. The theophylline tablet of Claim 1, 2, 3 or 4 wherein said bulkling agent is dibasic calcium phosphate.

5. A process for preparing a constant release rate theophylline tablet comprising the steps of dissolving a slow-dissolving, water-insoluble cellulose derivative in a solvent, admixing the resulting solution with a dry blend of anhydrous theophylline, and bulking agent to form a wet granulation, thoroughly drying said wet granulation, screening said dry granulation through a 14-20 mesh screen and compressing said sized granules.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 96, no. 2, 11th January 1982, page 336, no. 11678k, Columbus, Ohio, US; & JP - A - 81 122 311 (EISAI CO., LTD.) 25-09-1981 | | A 61 K 9/22 <br> A 61 K 31/52 |
| | --- | | |
| A | US-A-4 261 970 (OGAWA et al.) | | |
| | --- | | |
| A | US-A-4 259 314 (LOWEY) <br><br> * Column 5, example 3 * | | |
| | --- | | |
| A | GB-A-2 067 072 (THE BOOTS CO.) | | |
| | --- | | TECHNICAL FIELDS SEARCHED (Int. Cl. 3) |
| T | EP-A-0 109 320 (PIERRE FABRE) | | A 61 K |
| | ----- | | |

The present search report has been drawn up for all claims

| Place of search | Date of completion of the search | Examiner |
|---|---|---|
| THE HAGUE | 21-06-1984 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82